# EUROPEAN PATENT APPLICATION

(11) **EP 4 765 033 A1**
(43) Date of publication of application: **24.06.2026**
(21) Application number: 24307156.0
(22) Date of filing: 18.12.2024
(51) Int. Cl.: G06V 10/24, G06V 10/44, G06V 10/75, G06V 10/74, G06V 20/69, G06V 10/36, A61B 1/00

(54) **DETERMINING CORRESPONDENCES BETWEEN FEATURES OF A STRUCTURE IN A SEQUENCE OF IMAGES**

(71) Applicant: S.A. Vitec, 75008 Paris (FR)
(72) Inventor: AMESTOY, Thomas, 92320 CHATILLON (FR)
(74) Representative: Plasseraud IP

(57) **Abstract**

The disclosure relates to a method implemented by computer means for determining correspondences between features of a structure in a sequence of images, the method comprising:
obtaining a dynamic set of key points associated with positions in a reference image;
for each feature identified in a given image of the sequence:
determining (131, 151, 171, 191, 192) a correspondence between the feature and a reference key point of a set of reference key points based on a similarity criterion;
updating (210, 220, 310, 320, 330, 330, 331) the dynamic set of key points based on the determined correspondence and at least one predefined rule for assigning positions and associated values to the features; and
dynamically excluding (211) key points from the dynamic set of key points based on their associated values exceeding a predefined threshold.

## Description

### DOMAIN

The present invention relates to a method for determining correspondences between features of a structure in a sequence of images and to a corresponding system, a corresponding computer program and a corresponding computer-readable storage medium.

### BACKGROUND

In the field of computer vision, determining correspondences between features in a sequence of images is a critical step in various applications, such as object tracking, image alignment, and 3D reconstruction. Known methods often rely on feature detection and matching techniques using descriptors and similarity metrics. While these methods can be effective under ideal conditions, they face significant limitations, including:
sensitivity to changes in illumination, perspective, or occlusion,
propagation of errors when features are misidentified or descriptors fail to capture distinctive characteristics, and
inefficiencies in handling large image sequences or dynamic changes in the structure being analyzed.

These limitations can compromise the accuracy and reliability of applications that rely on robust feature correspondences. Consequently, there is a need for methods that can dynamically adapt to changing conditions while ensuring high precision in determining correspondences across image sequences.

### SUMMARY

The invention is defined by the appended independent claims. Additional features and advantages of the concepts herein disclosed are set forth in the description which follows.

The present disclosure aims at improving the situation.

To this end, the present disclosure describes a method implemented by computer means for determining correspondences between features of a structure in a sequence of images, the method comprising:
obtaining a dynamic set of key points associated with positions in a reference image;
for each feature identified in a given image of the sequence:
   determining a correspondence between the feature and a reference key point of a set of reference key points based on a similarity criterion;
   updating the dynamic set of key points based on the determined correspondence and at least one predefined rule for assigning positions and associated values to the features; and
   dynamically excluding key points from the dynamic set of key points based on their associated values exceeding a predefined threshold.

The proposed method introduces dynamic updating of the set of key points, which overcomes several limitations of the prior art.

By dynamically updating the set of key points, the method can adapt to changes in illumination, perspective, or occlusion, addressing the sensitivity of prior methods to these factors. For instance, as new features are detected and matched across frames, the dynamic set may evolve to reflect the current state of the structure, ensuring that the method remains robust even in dynamic environments.

The use of predefined rules to update positions and associated values may contribute that errors in matching do not propagate through the sequence. For example, unmatched features may be handled separately, limiting their impact on further correspondence determinations involving subsequent images of the sequence.

Dynamically excluding key points based on their associated values exceeding a predefined threshold provides additional advantages, including an improved reliability of the dynamic set, noise and outlier suppression, prevention of error accumulation, and improved resource allocation.

The present disclosure also describes a system comprising at least a processor operably connected to a computer-readable storage medium, the system being configured to determine correspondences between features of a structure in a sequence of images by: obtaining a dynamic set of key points associated with positions in a reference image; for each feature identified in a given image of the sequence:
determining a correspondence between the feature and a reference key point of a set of reference key points based on a similarity criterion;
updating the dynamic set of key points based on the determined correspondence and at least one predefined rule for assigning positions and associated values to the features; and
dynamically excluding key points from the dynamic set of key pointsbased on their associated values exceeding a predefined threshold.

The present disclosure also describes a computer-readable storage medium, optionally a non-transitory computer-readable storage medium, having stored thereon a computer program comprising instructions which, when executed by a processor, cause the processor to carry out the method hereby described.

The storage medium may include hard drives, solid-state drives, CDs, USB drives, etc. In this context, it is being used to refer to whatever medium is storing the computer program that runs the method.

The present disclosure also describes a computer program comprising instructions that are accessible to a processor and which, when executed by the processor, cause the processor to carry out the method hereby described.

The method described in the present disclosure may furthermore comprise the following features, taken alone or in combination.

In an example, determining the correspondence comprises determining that a feature in the given image matches a reference key point and identifying the feature in the given image as matched.

In an example, updating the dynamic set of key points comprises, upon identifying a feature in the given image as matched, assigning to the matched feature a position, in the reference image, assigned to the corresponding reference key point and an associated value determined based on a predefined relation with an associated value assigned to the corresponding reference key point.

In an example, determining the correspondence comprises:
determining that a feature in the given image does not match any reference key point of the set of reference key points,
determining that the feature in the given image matches a key point of the dynamic set of key points based on a similarity criterion, and
identifying the feature in the given image as matched.

In an example, updating the dynamic set of key points comprises, upon identifying a feature in the given image as matched, assigning to the matched feature a position in the reference image corresponding to an assigned position in the reference image of the corresponding dynamic key point and an associated value based on a predefined relation with an assigned associated value of the feature in the previously processed image.

In an example, the method further comprises, upon identifying a feature as matched, performing a reciprocal matching check before updating the dynamic set of key points.

In an example, determining the correspondence comprises:
determining that a feature in the given image does not match any reference key point of the set of reference key points and that the feature in the given image does not match any key point of the dynamic set of key points; and
identifying the feature in the given image as unmatched.

In an example, updating the dynamic set of key points comprises, upon identifying a feature in the given image as unmatched, assigning to the unmatched feature a position in the reference image interpolated from assigned positions in the reference image of neighboring matched key points of the dynamic set of key points and an associated value based on a predefined relation with associated values of the neighboring matched key points of the dynamic set of key points.

In an example, the position interpolated from the assigned positions in the reference image of the neighboring matched key points of the dynamic set of key points is determined using Delaunay triangulation.

In an example, the reference image is divided in a plurality of cells forming a grid, and updating the dynamic set of key points comprises maintaining a maximum of one key point in each cell.

In an example, updating the dynamic set of key points comprises populating the grid as features are identified and matched across the sequence of images.

In an example, updating the dynamic set of key points comprises replacing a previous key point with a new key point in the same cell based on a predefined rule.

In an example, determining the correspondences based on the similarity criterion comprises comparing descriptors representing local features of the structure.

In an example, the structure corresponds to biological tissue observed in fluorescence-based diagnostics, and the dynamic set of key points is used to align the images of the sequence.

### BRIEF DESCRIPTION OF THE DRAWINGS

Figure 1 depicts a system for determining correspondences between features of a structure in a sequence of images, in an exemplary embodiment.
Figure 2 depicts a workflow of a method for determining correspondences between features of a structure in a sequence of images, in an exemplary embodiment.
Figures 3, 4, 5 and 6 depict example portions of a workflow for managing a dynamic set of key points, in an exemplary embodiment
Figure 7 depicts a processing circuit, in an exemplary embodiment.

### DETAILED DESCRIPTION

The present disclosure is focused on a method for determining correspondences between features of a structure in a sequence of images.

A structure, in the context of the present disclosure, refers to a physical object or arrangement that can be captured in an image and analyzed for its characteristics. Examples include biological tissues or organs in the field of medical imaging, mechanical components in an industrial setup, landmarks or geographic features in aerial or satellite imagery, or any kind of item in a video surveillance context. The term "structure" is not limited to static objects and can include objects that change shape, position, or configuration over time (e.g., deformable organs, moving vehicles, rotating machinery, etc.)

An image refers to a representation of a scene or object captured by an imaging device. In the context of the disclosure, an image of the structure may be two-dimensional or three-dimensional. Examples of imaging devices may include visible light cameras, infrared sensors, fluorescence-based imaging systems, ultrasound sensors, X-ray sensors, radar, lidar, etc. An image may be two-dimensional (2D) or three-dimensional (3D) and typically consists of a grid of pixels. Each pixel is associated with one or more values representing image attributes such as intensity, color, or spectral information.

A frame refers to an individual image within a sequence of images, such as a video. Frames may represent temporally ordered snapshots of the structure, captured at specific time intervals, and are often synonymous with images in a sequence. In certain contexts, a frame may also denote a portion of an image or a region of interest (ROI) within a larger image, allowing for localized analysis of specific areas of the structure. For instance, in fluorescence-based imaging, a frame may correspond to an individual time step in the dynamic sequence showing changes in tissue fluorescence.

A sequence of images capturing a structure refers to a collection of images capturing the structure, the images of the sequence being ordered based on a temporal, spatial, or logical relationship. Examples may include video streams (temporal relationship), image slices (spatial relationship), or images from multiple cameras capturing the same scene (logical relationship). Temporal order is seen as the most common scenario, particularly in video or time-series imaging.

Features refer to specific, identifiable characteristics of a structure visible in an image. These may include points, edges, or regions of interest that are distinctive and can be described computationally. A feature may be represented for example by:
a position, for instance coordinates of the feature within the image, for instance in the form of coordinates of one or more pixels,
a descriptor, for instance a vector or a signature summarizing one or more local image characteristics of the feature, such as a gradient, a texture, and/or an intensity pattern, and
when appropriate, additional metadata such as a confidence score, a scale, an orientation, etc.

While a position and a descriptor are common characteristics of features in image processing, certain features might lack one or both in specific contexts. For instance, some features may be global or non-localized, such as an average brightness of a region or the overall texture pattern of an area. For instance, some features may be represented solely by their position, particularly when the application does not require detailed local characterization (e.g., a simple tracking of centroid points). Features of a structure may change position or appearance across a sequence of images, for instance due to motion, deformation, or occlusion.

Identifying a feature in an image involves detecting specific characteristics of the structure that meet predefined criteria for recognition. This may involve various computer vision techniques. Known feature detection algorithms include corner detection (e.g., Harris detector), edge detection (e.g., Canny operator), or blob detection (e.g., Laplacian of Gaussian). Features detected in an image may be filtered based on metrics like contrast, distinctiveness, or response intensity. Once a feature is identified in an image, the feature's position, descriptor, and optional metadata may be determined or extracted for further processing.

Key points are a subset of a set of features that are specifically selected for tracking or further analysis. Key points may be distinguished from features by their importance to a processing task and may be chosen based on their distinctiveness or robustness across the sequence of images.

A key point may be represented for example by:
a position in the reference image or in another image of the sequence,
a descriptor representing its local characteristics in the reference image, or in another image of the sequence, or aggregated across multiple images in the sequence,
one or more additional associated values, such as a drift value and/or a confidence score, and when appropriate, additional metadata.

Key points may be derived from features using a selection criterion, for example, prioritizing features with a high confidence score and/or a low drift value.

A reference image is an image selected from the sequence of images to serve as a fixed spatial reference for analyzing or tracking features. The reference image may be the first image in the sequence, or the reference image may be dynamically selected based on criteria such as image quality or relevance to a processing task. Key points may be initially defined relative to the reference image, with their positions and descriptors serving as baseline information for further analysis.

Correspondences between features of a structure in a sequence of images refer to pairs of features identified in at least two images of the sequence and that represent the same physical point of the structure or the same characteristic of the structure. Determining these correspondences involves computationally finding these pairs using a similarity criterion, such as a comparison of feature descriptors (e.g. Euclidean distance, cosine similarity, etc.) to evaluate a likelihood that two features "match", in the sense of "represent the same point of the structure or the same characteristic of the structure" or on the contrary, do not "match".

In computer vision, known methods for the processing task of determining correspondence between features of a structure in a sequence of image typically follow a multi-step approach which relies on identifying features in individual images and matching them across pairs of images using descriptors and similarity metrics.

While these methods can effectively establish correspondences under stable conditions, they exhibit significant limitations. They are sensitive to changes in illumination, perspective, or occlusion, making them less reliable for complex or dynamic sequences. They often require fixed, preselected sets of features that are not adapted to changes over time, leading to error propagation and reduced accuracy. They are computationally intensive when applied to large sequences, as they lack efficient mechanisms to dynamically manage feature sets. These limitations highlight the need for methods that can dynamically adapt to evolving conditions in the sequence while maintaining accuracy and computational efficiency.

To overcome these limitations, the present disclosure introduces the concept of a dynamic set of key points, which is incrementally updated to adapt to changes in the structure and sequence over time. This approach eliminates the static nature of prior art methods by dynamically managing the set of key points used for establishing correspondences.

A dynamic set of key points refers to a collection of key points that are initialized, updated, and refined over time as new images in the sequence are processed. The set is called "dynamic" because it is incrementally updated as features in new images are matched and evaluated. Key points may be added, replaced or excluded based on predefined rules and thresholds while processing the images in the sequence for determining the correspondences. Associated values, such as drift value and/or confidence score, may further evolve based on predefined rules while processing the images in the sequence for determining the correspondences.

By continuously adapting the set of key points, the present disclosure allows maintaining accuracy even under challenging conditions, such as motion, deformation, or occlusion. Further, the dynamic exclusion of unreliable key points may prevent the accumulation of errors, which is a common issue in static feature sets. Further, the use of predefined rules and thresholds for managing the dynamic set may reduce unnecessary comparisons and processing overhead.

In an exemplary aspect, the proposed technique is applied in the context of fluorescence imaging in a medical application.

In medical procedures, fluorescence imaging may help surgeons visualize biological tissues in real time. This technique involves injecting a fluorescent agent into the body, which then emits light when excited by a specific wavelength. The intensity of this fluorescence may be captured by an endoscope equipped with a camera, producing a sequence of images. By analyzing these images, medical professionals may gain valuable insights into tissue perfusion and functionality.

One important metric derived from such fluorescence imaging is the time to reach peak fluorescence, which indicates the time it takes for a particular region of tissue to reach its maximum fluorescence intensity after the agent is introduced. This metric is particularly useful for surgeons during procedures because it provides real-time feedback on tissue perfusion, it helps identify areas with poor or abnormal blood flow, and it may assist in making intraoperative decisions, such as assessing the viability of tissues or detecting abnormalities.

To make this information immediately actionable, an overlay may be applied to the live video stream captured by the endoscope. This overlay may take the form of a heatmap indicating the time to reach peak fluorescence for each pixel. The heatmap may be configured to evolve dynamically as the video progresses, allowing the surgeon to visualize perfusion patterns and respond accordingly.

Achieving this functionality imposes strict precision requirements and presents several technical challenges.

Regarding the precision requirements, the overlay should align with the live video as much as possible, preferably down to the pixel level. Even slight misalignments may lead to misleading visualizations, impacting the surgeon's ability to make accurate decisions.

Known challenges involve:
the length of image sequences, considering that a fluorescence imaging process may require analyzing and aligning hundreds or thousands of frames over time,
changes in illumination, due to changes in the imaging environment,
camera movement, due to the endoscope moving during the procedure and causing shifts in the field of view,
global body movement, due to the patient's breathing or other involuntary movements,
local organ movements such as shifts or deformations that may create complex spatial transformations, and
the surgeon's direct interaction with the tissue which may also introduce localized movements and deformations.

Existing methods for aligning images and generating overlays are inefficient and unreliable in some contexts, particularly when dealing with the challenges of uniform and deformable organs in real-time medical procedures. Several known techniques have been developed for image alignment and motion compensation, but they exhibit significant limitations when applied to the dynamic and complex conditions encountered during surgery.

One commonly used approach is motion estimation and compensation using fixed-size blocks, as seen in many video encoding and decoding algorithms. These algorithms divide each image into blocks of fixed dimensions and estimate the motion of each block to compensate for movement. While this approach is computationally efficient, it struggles to handle cases where multiple objects within a single block exhibit different motions, leading to inaccuracies in alignment. Additionally, motion estimation algorithms are particularly vulnerable to certain conditions, such as rotations, changes in lighting, and zoom effects, which can significantly degrade their performance. Furthermore, these algorithms are often optimized for entropy reduction rather than precise feature matching, which can result in suboptimal alignment for applications requiring high accuracy in feature preservation.

An improvement over fixed-size blocks involves variable-size block compensation, which allows block sizes to adapt to the motion dynamics of the scene. This technique can better handle heterogeneous motions but remains largely limited to translational movements. More complex transformations, such as rotations or scaling introduced by camera zoom or rotation, are not effectively addressed.

Optical flow is another well-known method that computes a dense field of motion vectors, one for each pixel in the image. This approach offers fine-grained motion compensation and can handle complex camera movements, such as rotations or zooms. However, optical flow is sensitive to changes in illumination and large movements, which are common in fluorescence imaging due to varying lighting conditions or tissue properties. Moreover, the cumulative error in pixels displacement, called drift, is difficult to control.

Another known technique is described in Szeliski, Richard, "Computer Vision: Algorithms and Applications" Springer Cham, 2022, https://doi.org/10.1007/978-3-030-34372-9 [Refl].

The technique in [Refl] proposes image alignment based on homographic transformations. This method uses a 3x3 homography matrix to map one image onto another, allowing for the correction of perspective, scaling, rotation, and translation. The approach is particularly effective for global camera movements and can produce an aligned sequence of images by sequentially mapping each frame to a fixed reference frame.

To estimate the coefficients of the homography matrix, [Refl] outlines the following steps:
a feature detection step,
a descriptor calculation step,
a matching step, and
a homography matrix coefficient estimation step.

The feature detection step involves detecting specific local features in both the reference image (Frame A) and the current image (Frame B) using algorithms such as GLAM points, as described in Truong, P., Apostolopoulos, S., Mosinska, A., Stucky, S., Ciller, C., & Zanet, S. D. (2019). Glampoints: Greedily learned accurate match points. In Proceedings of the IEEE/CVF International Conference on Computer Vision (pp. 10732-10741) [Ref2].

The descriptor calculation step involves computing descriptors for each detected feature using algorithms such as ORB (Oriented Fast and Rotated BRIEF), as described in E. Rublee, V. Rabaud, K. Konolige and G. Bradski, "ORB: An efficient alternative to SIFT or SURF," 2011 International Conference on Computer Vision, Barcelona, Spain, 2011, pp. 2564-2571, doi: 10.1109/ICCV.2011.6126544 [Refi],

The matching step involves matching descriptors between features in the two images using a K-nearest neighbor algorithm.

The homography matrix coefficient estimation step involves estimating the homography matrix coefficients based on matched descriptors.

This approach is robust to changes in illumination and can accurately compensate for global camera movements such as translations, rotations, and zooms. However, as noted in [Ref1], it has significant limitations when dealing with local movements, such as: deformations or shifts of tissues caused by physiological processes (e.g., breathing or organ movements), and
manipulations of tissues by the surgeon during the procedure.

These limitations reduce its effectiveness in contexts requiring precise, pixel-level alignment for dynamic and localized changes.

To address these limitations, the current disclosure introduces a method leveraging key points and features to automatically and dynamically align an overlay with the live video. This method builds on the homographic alignment approach described in [Refl], incorporating additional technical refinements that enable more robust handling of local deformations and movements, and enhanced real-time precision.

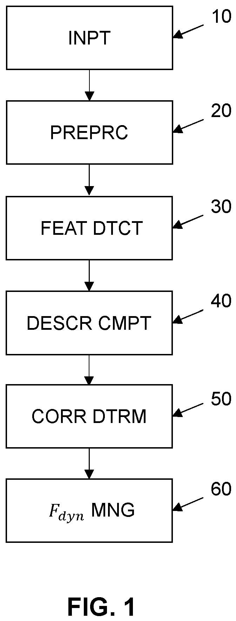

To facilitate understanding of the proposed technique, Figure 1 illustrates a system for determining correspondences between features of a structure in a sequence of images, in an exemplary embodiment.

The system comprises a series of functional modules, which may be implemented in software and/or hardware, each performing a specific operation in the process.

The modules are identified as follows.

An input module 10 is responsible for acquiring and managing the sequence of images showing the structure. The sequence of images may be obtained for instance from an image acquisition unit or imaging device (e.g., an endoscope or fluorescence imaging system) associated with a sequence management unit configured to organize the images in a temporal, spatial and/or logical order.

A preprocessing module 20 may be provided to prepare the images for further analysis by enhancing their quality and ensuring consistency. The preprocessing module may comprise a noise reduction unit for applying filters to reduce artifacts, and/or a normalization unit for adjusting brightness, contrast, or scale across images, among various possibilities.

A feature detection module 30 is configured to identify features in the images of the sequence, such as edges, corners, or regions of interest.

A descriptor computation module 40 is configured to compute descriptors for each identified feature or key point, summarizing their local image characteristics.

A correspondence determination module 50 is configured to compare descriptors of features in subsequent frames with descriptors of key points in the reference frame to establish correspondences. The correspondence determination module may comprise a similarity computation unit for calculating similarity scores (e.g., using Euclidean distance or cosine similarity) and a matching unit for determining matches based at least on the similarity scores and, possibly, further based on predefined thresholds.

A dynamic set of key points management module 60 is configured to manage a dynamic set of key points by initializing it in the reference frame and continuously updating it as subsequent frames are processed. The dynamic set of key points management module 60 may comprise for instance one or more of the following units (not represented on Figure 1):
a key point selection unit, which is configured to determine, upon processing a frame, which features identified in one or more images of the sequence are to be included in the dynamic set of key points,
a key point exclusion unit, which is configured to determine, upon processing a frame, which key points of the dynamic set of key points are to be excluded from the dynamic set of key points,
a position assignment unit, which is configured to assign and manage positions associated to the key points,
an associated value assignment unit, which is configured to assign and manage associated values (e.g. drift values or confidence scores) associated to the key points, and
a key point data storage unit which is configured to maintain records of key point data that are associated with the dynamic set of key points (e.g. positions, drift values, descriptors and other metadata) and to facilitate retrieval and update of the key point data.

The system may be configured to process a sequence of images received by the input module 10 and optionally preprocessed by the preprocessing module 20 in the following manner.

A reference frame #0 is initially selected in the sequence of images by the system (for instance by the dynamic set of key points management module 60). The reference frame may for instance be the first image of the sequence in the temporal order.

In the reference frame #0, a set of candidate features is identified by the feature detection module 30. These candidate features represent robust, distinctive features of the structure within the reference frame. From this initial set of candidate features, the dynamic set of key points management module 60 selects and stores certain features as key points in a dynamic set of key points, enabling them to serve as anchors for tracking features across the sequence of images.

Each key point in the dynamic set is assigned a position by the dynamic set of key points management module 60. The position of a key point within the reference frame is typically represented as the coordinates of the feature in the image grid (e.g., pixel coordinates).

The process of selecting key points from the set of candidate features, as well as initializing additional key points through interpolation, is described in detail below.

To ensure consistent distribution and coverage of key points across the reference image, the reference image may be conceptually divided into a plurality of cells forming a grid. For example, a cell may be *a* pixels wide and *b* pixels high, totaling *a*×*b* pixels, where *a* and *b* are both positive integers. An example configuration may be *a = b* = 16, forming cells of 256 pixels each. This grid-based division may help prevent clustering of key points in certain regions and ensures that key points are uniformly distributed across the image.

Candidate features detected by the feature detection module 30 may be retained as key points by the dynamic set of key points management module 60 based on one or more specific criteria. These criteria can be applied in isolation or in combination to determine which candidate features are selected as key points.

For instance, a distinctiveness criterion may be applied to evaluate the robustness or uniqueness of candidate features based on specific characteristics such as local contrast, separability in a descriptor space, edge strength, etc. Distinctiveness scores may be computed for each candidate feature, and the set of candidate features may be filtered to retain only those exceeding a predefined threshold.

For instance, a uniformity criterion may be applied to ensure a consistent density of key points across the reference image. In a grid-based configuration, this criterion seeks to achieve a uniform distribution of key points, for example by aiming to retain exactly one key point per cell, possibly with some tolerance. Several strategies may be employed for this selection. In an example strategy combining the uniformity criterion with the distinctiveness criterion, if multiple candidate features are detected within a single cell, the feature with the highest distinctiveness score may be selected as the key point for that cell. If no candidate features with sufficient distinctiveness scores are detected in a given cell, the cell may be flagged as empty for further processing (e.g., interpolation).

For instance, a spatial proximity criterion may be applied so that selected key points are not clustered too closely together. According to an example strategy, if multiple candidate features are present in the same grid cell or in adjacent grid cells, the dynamic set of key points management module 60 may be configured to select features that maximize the average pairwise distance between key points.

For instance, a temporal stability criterion may be applied to prioritize features that are likely to remain detectable across multiple frames. According to an example strategy, candidate features that appear consistently in a series of preliminary frames may be given higher priority when initializing the key points.

Cells without candidate features that satisfy the applied criterion or criteria may simply be left empty during the initialization of key points for the reference frame #0. In this case, interpolation methods, such as linear interpolation, Delaunay triangulation, weighted averaging, or radial basis functions (RBFs) are not applied to initialize key points in the reference frame #0. These methods may, however, be employed in subsequent frames or stages maintain uniform coverage across the image sequence.

One approach to combining multiple criteria is to define a cost function that assigns a numerical value to each candidate feature based on weighted contributions from different criteria. Candidate features with the highest cost function values are then selected to form the dynamic set of key points.

Heuristic rules may also be defined to prioritize certain criteria in specific contexts. For example, for regions near the center of the image, distinctiveness may be prioritized (e.g., to track stable features for global alignment), whereas for peripheral regions, uniformity may be prioritized (e.g., to ensure coverage across the entire image).

Another approach involves applying a decision tree, where each criterion is evaluated sequentially. An exemplary strategy may be described as following:
if the cell contains only one candidate feature, select it as the key point, else
if multiple candidate features are detected, select the candidate feature with the highest distinctiveness score, else
if distinctiveness scores are tied, apply the spatial proximity criterion to break the tie, else
if the cell is empty, proceed to interpolation.

After initialization, the set of key points then comprises, at least, key points selected directly from the set of candidate features in the reference frame #0 based on the applied criterion / criteria. The set of key points may further comprise interpolated key points.

For each key point in the dynamic set, the dynamic set of key points management module 60 stores the following data:
a position,
a descriptor, and
one or more associated values.

The position is represented for instance as pixel coordinates in the reference frame, e.g. (x, y) where x and y correspond to the horizontal and vertical indices of the pixel in the reference frame.

The descriptors for the key points may be determined according to one or more possible modes, depending on factors such as computational resources, a desired level of precision, or specific application requirements.

For each key point selected from the set of candidate features in the reference frame #0, a descriptor is computed by the descriptor computation module 40 according to a first mode, in a known manner. The computation according to the first mode may for instance involve analyzing the local image characteristics around the key point, such as gradients, textures, or intensity distributions, using established methods (e.g., ORB, SIFT, or other descriptor algorithms).

For each interpolated key point, a default descriptor may be assigned according to a second mode instead of computing a descriptor from the image according to the first mode. The default descriptor may represent a neutral or average value that facilitates downstream processing without biasing the interpolation-based key points as overly distinct or important.

Alternatively, the descriptor computation module 40 may be configured to compute a descriptor for every key point in the dynamic set and the dynamic set of key points management module 60 may be configured to store the computed descriptors regardless of how the corresponding key point was derived.

The drift values for the key points quantify the estimated cumulative error in displacement of the key points relative to their original position in the reference frame.

When initializing the dynamic set of key points in the reference frame, their drift values may all be fixed to the same predefined default value, such as 1, indicating no displacement.

Alternatively, when initializing the dynamic set of key points in the reference frame, the drift values may be assigned distinct predefined default values depending on whether the key point was selected from the set of candidate features or interpolated. For key points selected from the set of candidate features, the drift value may be fixed to a first default value, such as 1, whereas for interpolated key points, the drift value may be fixed to a different default value, such as 2, representing an initial status that accounts for the fact that the position of the key point was derived through interpolation rather than direct detection.

According to a possible convention, increasing drift values indicate cumulative error in displacement over time as subsequent frames are processed. The drift value of a key point may be incremented as the key point is updated, reflecting its ongoing motion relative to its original position in the reference frame. Distinct initial drift values for interpolated and non-interpolated key points may aid in distinguishing their origins during downstream analysis or updates.

After the reference frame has been processed and the dynamic set of key points is initialized, subsequent frames are processed one by one and the dynamic set of key points is updated as the subsequent frames are processed.

The processing of each subsequent frame (#i) involves:
identifying features and computing their descriptors,
comparing features with reference key points in the reference frame to establish correspondences,
handling matched features by assigning positions and drift values based on predefined rules, and
handling unmatched features through secondary comparisons with key points of the dynamic set, interpolation, or exclusion, as appropriate.

The process of updating the set of key points is described in detail below.

In each subsequent frame #i after the reference frame #0, features of the structure are identified by the feature detection module 30. These features may correspond to edges, corners, or other distinctive characteristics of the structure visible in the image.

For each feature in the current frame #i, a descriptor is computed by the descriptor computation module 40 using the same method applied to the key points in the reference frame #0.

To ensure robustness against variations in imaging conditions, the descriptors may be normalized to account for differences in illumination, contrast, or scale. Additionally, similar to reference key points in the reference frame, the features in the current frame may be associated with metadata, such as confidence scores or quality indicators, which can inform downstream processing steps.

The descriptors of features in the current frame #i are compared with the descriptors of reference key points in the reference frame #0 using a similarity criterion, such as Euclidean distance, cosine similarity, or other distance metrics. This comparison determines correspondences between features in the current frame and reference key points in the reference frame.

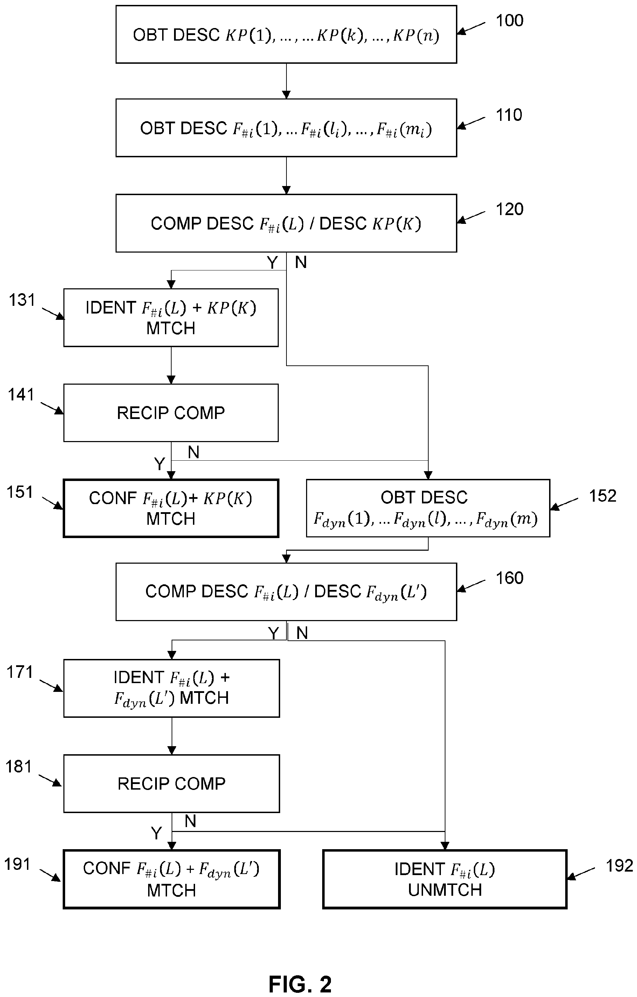

A possible workflow for determining the correspondences is now described, in relation with Figure 2.

Let us denote:
a set of *n* reference key points *KP* in the reference frame as *KP*(1), ..., ... *KP(k),* ..., *KP*(*n*), where each key point has an index *k*,
a set of *mᵢ* features *F* in the current frame #i as *F_{#i}*(1), ... *F_{#i}*(*lᵢ*), ... , *F_{#i}*(*mᵢ*), where each feature has an index *lᵢ*, and
a dynamic set of key points *F_{dyn}* as *F_{dyn}*(1), ... *F_{dyn}*(*l*), ... , *F_{dyn}*(*m*), where each key point of the set has an index *l*.

*KP* and *F_{dyn}* may both be initialized upon processing the reference frame as being identical to each other.

*F_{dyn}* may be updated upon processing any frame that is not the reference frame, based on applying at least one predetermined rule, as part of the possible workflow described herein.

It is considered that, upon initialization of *KP* and *F_{dyn},* each reference key point and each key point of the dynamic set of key points is assigned a descriptor, a position in the reference frame, and a default drift value (for instance 0).

It is further considered that when *F_{dyn}* is updated, the updating process may comprise one or more of the following:
adding one or more new key points to *F_{dyn},* each new key point being assigned a corresponding descriptor, a corresponding position in the reference frame, and a corresponding drift value,
deleting one or more key points from *F_{dyn},* and
modifying the descriptor and/or the position in the reference frame and/or the drift value of one or more key points of *F_{dyn}.*

It is further considered that *KP* is maintained as a set of static key points, in other words as reference key points that are not updated after the initialization of *KP.* This means that after the initialization of *KP,* no provision is made to:
add any new reference key points to *KP,*
delete one or more reference key points from *KP,* or
modify the descriptor and/or the position in the reference frame and/or the drift value of one or more reference key points of*KP.*

Let us further consider an arbitrary feature, *F_{#i}*(*L*), from the set of features in the current frame #i.

At block 100, the descriptors of *KP*(*1*), ..., ... *KP(k),* ..., *KP*(*n*) are obtained.

At block 110, the descriptors of *F_{#i}*(1), ... *F_{#i}*(*lᵢ*), ..., *F_{#i}*(*mᵢ*) are obtained.

At block 120, the descriptor of *F_{#i}*(*L*) may be compared with the descriptors of *KP*(*1*), ..., ... *KP*(*k*), ..., *KP*(*n*) using the similarity criterion. The comparison yields a set of similarity scores, where each score represents the likelihood of correspondence between *F_{#i}*(*L*) and a specific reference key point *KP(k).*

The reference key point *KP(K)* which has the highest similarity score may be identified at block 131 as a candidate match for *F_{#i}*(*L*), provided this similarity score exceeds a predefined threshold.

To confirm the correspondence, a reciprocal check may be conducted at block 141. The descriptor of *KP(K)* may then be compared with the descriptors of *F_{#i}*(1), ... *F_{#i}*(*lᵢ*), ... , *F_{#i}*(*mᵢ*) using the similarity criterion. The comparison yields a set of similarity scores, where each score represents the likelihood of correspondence between *KP(K)* and a specific feature *F_{#i}*(*lᵢ*) in the current frame. If *F_{#i}*(*L*) also has the highest similarity score, the correspondence may be confirmed at block 151.

Matches are established when a correspondence is identified or confirmed between a feature in the current frame and a reference key point in the reference frame.

In a scenario, *F_{#i}*(*L*) is identified at block 131 and confirmed at block 151 as a matched feature and is associated with *KP(K)* which is identified and confirmed as its matching reference key point.

When no match is found between a feature *F_{#i}*(*L*) in the current frame and any reference key point in the reference frame, the feature is considered unmatched. To avoid discarding unmatched features prematurely, a secondary comparison may be performed.

Specifically, the descriptor of the unmatched feature *F_{#i}*(*L*) in the current frame #i may be compared with the descriptors of the key points *F_{dyn}*(1), ... *F_{dyn}*(*l*), ... , *F_{dyn}*(*m*)*.* The same similarity criterion (e.g., Euclidean distance, cosine similarity) may be used for this comparison.

In this case, at block 152, the descriptors of *F_{dyn}*(1), ... *F_{dyn}*(*l*), ..., *F_{dyn}*(*m*)are obtained and the comparison between the descriptor of the unmatched feature *F_{#i}*(*L*) in the current frame #i and the descriptors of *F_{dyn}*(1), *... F_{dyn}*(*l*), ... , *F_{dyn}*(*m*) is performed at block 160.

A reciprocal check may be conducted at block 181 to reduce the risk of incorrect matching.

A purpose of the secondary comparison is to ensure that key points of *F_{dyn}* which do not directly correspond to reference key points in the reference frame are not ignored. It facilitates tracking of features undergoing gradual or complex motions, such as deformations, rotations, or translations across the sequence.

In a scenario, *F_{#i}*(*L*) is identified at block 171 and confirmed at block 191 as a matched feature associated with *F_{dyn}*(*L'*) which is identified at block 171 and confirmed at block 191 as its matching key point of *F_{dyn}.*

When *F_{#i}*(*L*) is identified (or confirmed) as matched with a matching reference key point *KP(K)* or with a matching key point *F_{dyn}*(*L'*) of *F_{dyn},* the dynamic set of key points is updated to account for the matched feature *F_{#i}*(*L*).

To do so, specific data, such as a position in the reference frame, a drift value and a descriptor, may be assigned to the matched feature *F_{#i}*(*L*) based on one or more predefined rules. These specific data are designed to ensure that the matched feature can seamlessly integrate into the dynamic set of key points for further tracking and/or further analysis.

If no match is found even during the secondary comparison, *F_{#i}*(*L*) is identified as unmatched at block 192.

An unmatched feature may be interpolated to determine its position in the reference frame. Interpolation methods such as Delaunay triangulation may use neighboring matching key points to approximate the position of the unmatched feature. Alternatively, the unmatched feature may be excluded from further analysis based on predefined rules, such as confidence thresholds or drift value limits.

In the workflow described in Figure 2, three distinct conclusions may be reached when seeking correspondence between *F_{#i}*(*L*) and the reference frame:
*F_{#i}*(*L*) may be confirmed as matched with a reference key point, or
*F_{#i}*(*L*) may be confirmed as matched with a key point of *F_{dyn},* or
*F_{#i}*(*L*) may be identified as unmatched.

When the workflow is modified so that no reciprocal comparisons are conducted at blocks 141, 181, the three distinct conclusions are modified to:
*F_{#i}*(*L*) may be identified as matched with a reference key point, or
*F_{#i}*(*L*) may be identified as matched with a key point of *F_{dyn},* or
*F_{#i}*(*L*) may be identified as unmatched.

For a given workflow, the set of possible distinct conclusions are collectively denoted as a determined correspondence between *F_{#i}*(*L*) and the reference frame.

For each case, specific rules may be applied to update the dynamic set of key points to reflect the determined correspondence.

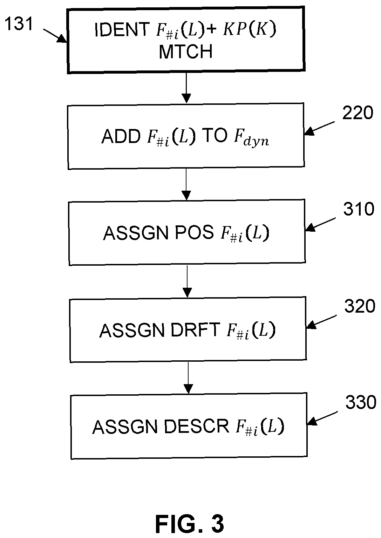

Figure 3 depicts an example workflow for the case where the determined correspondence is an identification at block 131 (or equivalently a confirmation at block 151) of *F_{#i}*(*L*) as matched with a reference key point *KP(K).*

The following updates and assignments may be made:
the dynamic set of key points *F_{dyn}* retains *F_{#i}*(*L*) as a key point at block 220,
the descriptor of *F_{#i}*(*L*) is assigned to *F_{#i}*(*L*) as key point of *F_{dyn}* at block 330,
the position of *KP(K)* in the reference frame is assigned to *F_{#i}*(*L*) as key point of *F_{dyn}* at block 310, and
the drift value of *KP(K)* is assigned to *F_{#i}*(*L*) as key point of *F_{dyn}* at block 320 (no drift introduced).

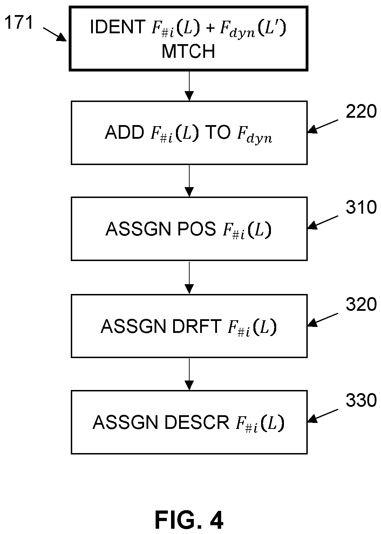

Figure 4 depicts an example workflow for the case where the determined correspondence is an identification at block 171 (or equivalently a confirmation at block 191) of *F_{#i}*(*L*) as matched with a key point *F_{dyn}*(*L'*)*.*

Similarly as in Figure 3, the following updates and assignments may be made: *F_{#i}*(*L*) is retained at block 220 as a key point of*F_{dyn},*
the position in the reference frame assigned to *F_{#i}*(*L*) as matched feature is assigned at block 310 to *F_{#i}*(*L*) as key point of *F_{dyn},*
a drift value is assigned at block 320 to *F_{#i}*(*L*) as key point of *F_{dyn},* and
the descriptor of *F_{#i}*(*L*) is retained and assigned at block 330 to *F_{#i}*(*L*) as key point of *F_{dyn}.*

The position in the reference frame assigned to *F_{#i}*(*L*) as key point of *F_{dyn}* may be determined for instance by propagating a position in the reference frame previously assigned to *F_{dyn}*(*L'*)*.*

The drift value *dᵢ* assigned to *F_{#i}*(*L*) as key point of *F_{dyn}* may be determined by applying a predefined relation which accounts for a drift value previously assigned to *F_{dyn}*(*L'*). For instance, the drift value *dᵢ* may be calculated as *dᵢ = dⱼ* + *k,* where *dⱼ* is the drift value previously assigned to*F_{dyn}*(*L'*), and *k* is a predefined, non-zero, offset. In this relation, the predefined offset k accounts for the incremental nature of displacement across frames, reflecting the cumulative drift of a feature as it is tracked over time. A simple value such as *k* = 1, may be used to simplify calculations. Alternatively, *k* may be adjusted based on specific motion dynamics, such as gradual or abrupt changes in the structure's movement.

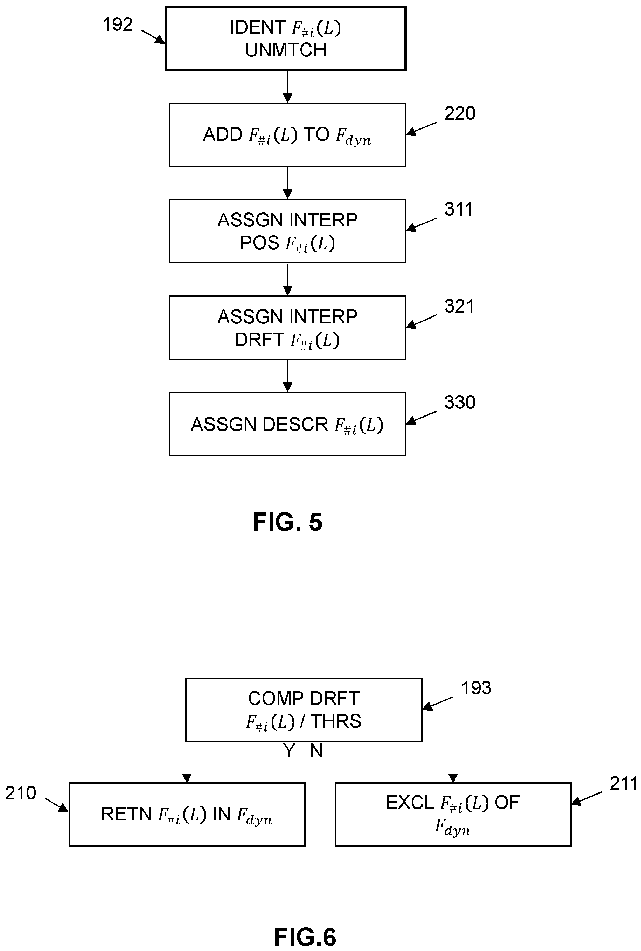

Figure 5 depicts an example workflow for the case where the determined correspondence is an identification at block 192 of *F_{#i}*(*L*) as unmatched, (i.e., no correspondence is found with any reference key point or any key point of *F_{dyn}*)*.*

The following updates and assignments may be made:
*F_{#i}*(*L*) may be added at block 220 to the dynamic set of key points as a key point,
an interpolated position in the reference frame is assigned at block 311 to *F_{#i}*(*L*) as key point of *F_{dyn},*
a default drift value or an interpolated drift value is assigned at block 321 to *F_{#i}*(*L*) as key point of *F_{dyn},* and
the descriptor of *F_{#i}*(*L*) is retained and assigned at block 330 as the descriptor of *F_{#i}*(*L*) as key point of *F_{dyn}*.

The interpolated position in the reference frame is determined for *F_{#i}*(*L*) using an interpolation method based on neighboring key points of *F_{dyn}* that are matching key points of other features of frame #i.

A default drift value (e.g., 2) may be assigned to *F_{#i}*(*L*), reflecting that its position was derived through interpolation rather than direct detection or matching. Alternatively, the drift value assigned to *F_{#i}*(*L*) may be computed using an interpolation technique similar to that used for position assignment.

For instance, an interpolation method such as Delaunay triangulation may be used to determine a position for *F_{#i}*(*L*) in the reference frame. This method identifies the three closest neighboring key points of *F_{dyn},* denoted as *k*₁, *k*₂ and *k*₃, based on their positions in the reference frame. These neighboring key points form a triangle within the Delaunay triangulation mesh that encloses the position of *F_{#i}*(*L*) in the current frame. The position assigned to *F_{#i}*(*L*) in the reference frame is then calculated as a weighted average of the positions of *k*₁, *k*₂ and *k*₃, where the weights are determined by the relative spatial proximity of *F_{#i}*(*L*) to each of the three neighboring key points in the current frame.

Continuing this example, the drift value *dᵢ* assigned to *F_{#i}*(*L*) may also be interpolated based on the drift values previously assigned to *k*₁, *k*₂ and *k*₃. Specifically, the drift value *dᵢ* can be calculated as *dᵢ* = f(*d*_{*k*₁}, *d*_{*k*₂}*, d*_{*k*₃}) + *k*, where *d*_{*k*₁}, *d*_{*k*₂}*, d*_{*k*₃} are the drift values previously assigned to key points *k*₁, *k*₂ and *k*₃, respectively, and *k* is a predefined offset (for instance *k* = 2) reflecting the incremental nature of drift across frames. The function f may, for instance, compute a weighted average of the drift values of *k*₁, *k*₂ and *k*₃, using the same weights as those applied during position interpolation. This approach ensures that the drift value of *F_{#i}*(*L*) accurately reflects the cumulative displacement of its interpolated position relative to the reference frame.

Alternatively, *F_{#i}*(*L*) may not be added to the dynamic set and may be discarded.

In addition to the mechanisms describing how to maintain a key point in the dynamic of set of key points, and how to add a new key point in the dynamic set of key points, further mechanisms may be provided to exclude key points or features that are no longer relevant or reliable.

Figure 6 depicts an example workflow of a mechanism for excluding key points by applying an exclusion criterion based on drift value.

As already explained, each key point has a drift value which initially depends on how the key point was added to the dynamic set:
upon initializing the dynamic set, or
as a result of a feature of a frame #i being matched with a corresponding reference key point or with a corresponding key point of the dynamic set, or
as a result of an interpolation.

As further frames are processed, for each key point that fails to be matched with a feature of the current frame, the drift value associated to this key point may further be incremented by a predefined offset (for instance by an offset equal to 1).

While a frame is being processed, or after a frame is processed, the drift values assigned to the key points *F_{dyn}*(1), ... *F_{dyn}*(*l*), ... , *F_{dyn}*(*m*) of the dynamic set may be compared at block 193 with a threshold (e.g. a value of 5 may serve as such a threshold, as indicating significant cumulative displacement and/or reduced reliability).

A key point *F_{dyn}*(*L'*) of the dynamic set of key points is then excluded at block 211 if its drift value exceeds the threshold. Conversely, the key point *F_{dyn}*(*L*') is retained at block 210 in the dynamic set of key points if its drift value does not exceed the threshold

Upon exclusion, the key point is removed from the dynamic set. Its associated data (position, descriptor, drift value) may no longer be made available for downstream processing.

A similar workflow as in Figure 6 may apply to filter features of frame #i based on the exclusion criterion in view of adding only the filtered features to the dynamic set of key points.

In experiments conducted by the inventor, applying the workflows described in Figures 2 to 6 to the processing of an image sequence used in fluorescence-based diagnostics, the proposed technique demonstrated a performance improvement compared to the algorithm described in [Refl], wherein to estimate the coefficients of the homography matrix, [Refl] outlines the following steps: a feature detection step (GLAM points), a descriptor calculation step (ORB), a matching step (K-Nearest Neighbors) and a homography matrix coefficient estimation step (RANSAC on matched KP). This performance improvement is attributed primarily to the use in the proposed technique of a dynamically updated set of key points.

Three specific types of scenarios illustrate the nature and extent of the performance improvements.

In scenarios with minimal global movement, such as limited camera or body motion, and minimal local movement, including reduced manipulation of organs or spontaneous deformations, the proposed technique results in an improvement of approximately +20% in the number of matches between key points when compared to the method outlined in [Refl].

In scenarios with significant global movement such as substantial camera or body motion, and significant local movement, including extensive manipulation of organs or spontaneous deformations, the proposed technique achieves a remarkable enhancement in the number of matches, ranging from +100% (2×) to +900% (10×) compared to the method in [Refl].

In scenarios with challenging conditions, such as significant changes in lighting or substantial zoom variations, the proposed technique demonstrates an improvement in the number of matches by a factor of +100% (2×) to +400% (5×) over the method described in [Ref1].

The strategies described above represent a primary workflow for updating the dynamic set of key points. Additional mechanisms may be provided, and alternative strategies may also be considered, depending on application-specific requirements.

For instance, one or more possible additional mechanisms may ensure that, assuming a grid structure, a specific density of key points per cell is maintained. A common approach is to enforce a maximum of one key point per cell to ensure uniform coverage across the reference frame.

When updating the dynamic set of key points, the grid may be populated as features are identified and matched across the sequence of images.

Specifically, a previous key point *F_{dyn}*(*L'*) in a cell may be replaced by a feature *F_{#i}*(*L*) of the current frame #i based on predefined conditions.

For instance, *F_{#i}*(*L*) may replace *F_{dyn}*(*L'*)*,* as being a more recent key point, if the position assigned to *F_{#i}*(*L*) in the reference frame falls within the same cell as *F_{dyn}*(*L'*)*.* An additional condition may be provided for the replacement, for instance, the additional condition may require that the drift value assigned to *F_{#i}*(*L*) is smaller than the drift value of *F_{dyn}*(*L'*)*.* The replacement process involves excluding *F_{dyn}*(*L'*) from the dynamic set and adding *F_{#i}*(*L*) as a new key point with its assigned position in the reference frame, assigned drift value, and descriptor.

If a key point *F_{dyn}*(*L'*) is excluded and no feature from the current frame #i satisfies the conditions for replacement, the cell may become void (i.e., without any key point). A mechanism may prioritize the addition of a new key point in the void when the next frame #i+1 is processed. This mechanism may include a void detection step, identifying cells without key points, and a priority assignment step, which gives features in void cells a higher likelihood of being selected as key points when features are identified in the subsequent frame. Features identified in the frame #i+1 and which are assigned positions falling within void cells of the reference frame may be favored as candidate key points, based on predefined rules, such as distinctiveness scores or confidence thresholds. Alternatively, as already described, interpolated key points may be generated for the void cells, using neighboring matching key points.

Although the grid structure provides a convenient way to ensure spatial uniformity and manage key points, alternative approaches may be employed:

Instead of relying on a grid, key points may be managed by analyzing pairwise distances between existing key points in the dynamic set. A predefined minimum distance may be enforced, ensuring that no two key points are closer than a specified threshold. If a new key point *F_{#i}*(*L*) is proposed, it may be excluded or replace an existing key point if it violates this minimum distance criterion.

A density metric may be computed based on the number of key points within a given radius or region of the reference frame. Key points may be added, excluded, or replaced to maintain a consistent density. For example, regions with too many key points may trigger exclusion based on drift values or confidence scores. For example, regions with too few key points may prioritize the addition of new key points during processing of subsequent frames.

Key points may be organized into clusters instead of a grid. Clusters may adapt dynamically to motion or deformation, ensuring that key points are grouped logically based on spatial proximity and drift values. New features may be assigned to clusters or trigger the creation of new clusters, depending on their location and characteristics.

Mechanisms may also be provided to handle scenarios where the dynamic set of key points requires a complete reset or when certain frames need to be ignored.

A reset may be triggered if the drift values of a significant portion of the key points exceed a threshold, indicating that the current reference frame no longer provides a stable anchor for tracking. In this case, a new reference frame is selected (e.g., the current frame), and the dynamic set is re-initialized using the detected features in the new reference frame.

A frame may be ignored entirely if it fails to meet predefined criteria (e.g., low image quality, excessive motion blur, or incomplete data). Features detected in the ignored frame are not processed, and the dynamic set remains unchanged.

Examples of possible alternative strategies are now described.

In a simplified approach, drift values may be omitted entirely, and only positions and descriptors are updated, simplifying the data structure and reducing computational complexity. Key points may be replaced or excluded purely based on confidence scores or distinctiveness, without tracking cumulative drift.

In a more complex approach, a weighted scoring system may be used to prioritize key points based on multiple factors, such as drift value, confidence score, and matching frequency. Dynamic clustering algorithms may be applied to group key points and ensure that updates maintain spatial coherence across the image.

In a hybrid approach, key points may be updated using different rules depending on the region of the image (e.g., high-motion vs. low-motion areas). The dynamic set may include a fixed quota of interpolated key points to fill gaps in coverage while prioritizing directly matched features.

Unless specifically stated otherwise, as apparent from the preceding discussions, it is appreciated that in the methods and workflows described herein, the use of terms such as "computing", "calculating", "generating", or the like, refer to the action and/or processes of a computer or computing system, or similar electronic computing device, that manipulate and/or transform data represented as physical, such as electronic, quantities within the computing system's registers and/or memories into other data similarly represented as physical quantities within the computing system's memories, registers or other such information storage, transmission or display devices.

Embodiments of the present invention may include apparatuses for performing the operations herein. This apparatus may be specially constructed for the desired purposes, or it may comprise a general-purpose computer or Digital Signal Processor ("DSP") selectively activated or reconfigured by a computer program stored in the computer. Such a computer program may be stored in a computer readable storage medium, such as, but is not limited to, any type of disk including floppy disks, optical disks, CD-ROMs, magnetic-optical disks, read-only memories (ROMs), random access memories (RAMs) electrically programmable read-only memories (EPROMs), electrically erasable and programmable read only memories (EEPROMs), magnetic or optical cards, or any other type of media suitable for storing electronic instructions, and capable of being coupled to a computer system bus.

The processes presented herein are not inherently related to any particular computer or other apparatus. Various general-purpose systems may be used with programs in accordance with the teachings herein, or it may prove convenient to construct a more specialized apparatus to perform the desired method. The desired structure for a variety of these systems will appear from the description below. In addition, embodiments of the present invention are not described with reference to any particular programming language. It will be appreciated that a variety of programming languages may be used to implement the teachings of the inventions as described herein.

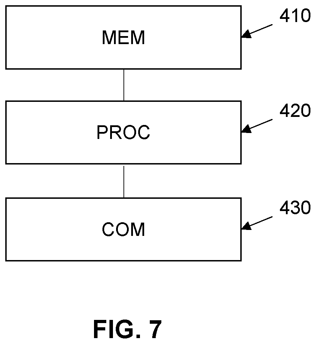

Figure 7 provides a generic representation of apparatuses and systems for performing the operations herein, in the form of a processing circuit comprising at least one computer-readable medium 410, operably connected to at least one processing unit 420 understood as a physical or virtual processor comprising any number of cores, e.g. CPU, GPU, ASIC, FPGA, etc., and that may be further operably connected to one or more input and/or output communication interfaces 430.

This general description is intended to present an exemplary implementation of the proposed technique. Variations, modifications, and alternatives may be apparent to those skilled in the art and can be made without departing from the scope of the invention. The specific configuration of components and the manner in which they interact are merely illustrative, and alternative configurations and interactions are within the scope of the appended claims.

The proposed technique finds immediate application in fluorescence-based diagnostics. During medical procedures, fluorescence imaging allows surgeons to visualize tissue perfusion in real time. The invention enables precise image alignment, ensuring that dynamic overlays, such as heatmaps indicating the time to reach peak fluorescence, align accurately with the live video feed from an endoscope. This precision facilitates intraoperative decision-making, such as assessing tissue viability or identifying regions of abnormal perfusion.

Beyond fluorescence-based diagnostics, the proposed technique may be applied in various technical fields using workflows that require precise alignment of image sequences, including video stabilization, remote sensing or aerial imaging.

The proposed technique may extend to use cases beyond image alignment, including mosaicking (for instance in geospatial analysis), estimation of position and/or orientation of objects (for instance in robotics, autonomous vehicles and augmented reality), object tracking (for instance in traffic surveillance), or 3D reconstruction (for instance in engineering or in virtual reality).

## Claims

1. A method implemented by computer means for determining correspondences between features of a structure in a sequence of images, the method comprising:
obtaining a dynamic set of key points associated with positions in a reference image;
for each feature identified in a given image of the sequence:
determining (131, 151, 171, 191, 192) a correspondence between the feature and a reference key point of a set of reference key points based on a similarity criterion;
updating (210, 220, 310, 320, 330, 330, 331) the dynamic set of key points based on the determined correspondence and at least one predefined rule for assigning positions and associated values to the features; and
dynamically excluding (211) key points from the dynamic set of key points based on their associated values exceeding a predefined threshold.

2. The method of the preceding claim, wherein determining the correspondence comprises determining that a feature in the given image matches a reference key point and identifying (131, 151) the feature in the given image as matched.

3. The method of the preceding claim, wherein updating the dynamic set of key points comprises, upon identifying a feature in the given image as matched, assigning to the matched feature a position, in the reference image, assigned to the corresponding reference key point and an associated value determined based on a predefined relation with an associated value assigned to the corresponding reference key point.

4. The method of any one of the preceding claims, wherein determining the correspondence comprises:
determining that a feature in the given image does not match any reference key point of the set of reference key points ,
determining that the feature in the given image matches a key point of the dynamic set of key points based on a similarity criterion, and
identifying (171, 191) the feature in the given image as matched.

5. The method of the preceding claim, wherein updating the dynamic set of key points comprises, upon identifying a feature in the given image as matched, assigning (310, 320) to the matched feature a position in the reference image corresponding to an assigned position in the reference image of the corresponding dynamic key point and an associated value based on a predefined relation with an assigned associated value of the corresponding dynamic key point.

6. The method of any one of claims 2 to 5, further comprising, upon identifying a feature as matched, performing a reciprocal matching check (141, 181) before updating the dynamic set of key points.

7. The method of any one of the preceding claims, wherein determining the correspondence comprises:
determining that a feature in the given image does not match any reference key point of the set of reference key points and that the feature in the given image does not match any key point of the dynamic set of key points; and
identifying (192) the feature in the given image as unmatched.

8. The method of the preceding claim, wherein updating the dynamic set of key points comprises, upon identifying a feature in the given image as unmatched, assigning (311, 321) to the unmatched feature a position in the reference image interpolated from assigned positions in the reference image of neighboring matched key points of the dynamic set of key points and an associated value based on a predefined relation with associated values of the neighboring matched key points of the dynamic set of key points.

9. The method of the preceding claim, wherein the position interpolated from the assigned positions in the reference image of the neighboring matched key points of the dynamic set of key points is determined using Delaunay triangulation.

10. The method of any one of the preceding claims, wherein the reference image is divided in a plurality of cells forming a grid, and wherein updating the dynamic set of key points comprises maintaining a maximum of one key point in each cell.

11. The method of the preceding claim, wherein updating the dynamic set of key points comprises populating the grid as features are identified and matched across the sequence of images.

12. The method of any one of the preceding claims, wherein updating the dynamic set of key points comprises replacing a previous key point with a new key point in the same cell based on a predefined rule.

13. The method of any one of the preceding claims, wherein determining the correspondences based on the similarity criterion comprises comparing (120, 160) descriptors representing local features of the structure.

14. The method of any one of the preceding claims, wherein the structure corresponds to biological tissue observed in fluorescence-based diagnostics, and the dynamic set of key points is used to align the images of the sequence.

15. A computer program product comprising instructions stored on a non-transitory computer-readable medium, the instructions, when executed by a processor, causing a system to perform a method for determining correspondences between features of a structure in a sequence of images, the method comprising:
obtaining a dynamic set of key points associated with positions in a reference image;
for each feature identified in a given image of the sequence:
determining (131, 151, 171, 191, 192) a correspondence between the feature and a reference key point of a set of reference key points based on a similarity criterion;
updating (210, 220, 310, 320, 330, 330, 331) the dynamic set of key points based on the determined correspondence and at least one predefined rule for assigning positions and associated values to the features; and
dynamically excluding (211) key points from the dynamic set of key points based on their associated values exceeding a predefined threshold.
